# EUROPEAN PATENT SPECIFICATION

(11) **EP 4 037 647 B1**
(45) Date of publication and mention of the grant of the patent: **12.08.2026**
(21) Application number: 20800495.2
(22) Date of filing: 13.10.2020
(51) Int. Cl.: A61K 8/41, A61K 8/02, A61K 8/73, A61Q 5/12

(54) **FILM COMPOSITIONS**
FILMZUSAMMENSETZUNGEN
COMPOSITIONS DE FILMS

(30) Priority: 25.10.2019 US 201962926099 P
(43) Date of publication of application: 10.08.2022
(73) Proprietor: Colgate-Palmolive Company, New York, NY 10022 (US)
(72) Inventor: JHA, Brajesh, Midlothian, Virginia 23112 (US); AGBLEY, Esinam Kuma, Somerset, New Jersey 08873 (US); ZUNIGA, Arturo, CP 11520 Del Miguel Hidalgo DF (MX)
(74) Representative: Wichmann, Hendrik
(86) International application number: PCT/US2020/055345
(87) International publication number: WO 2021/080814

(56) References cited:
- WO-A2-2009/006218
- DE-A1- 102010 027 487
- DE-U1- 202016 007 571

## Description

### BACKGROUND

Most individuals buy and use a hair shampoo for its cleansing properties, however, present-day hair shampoos generally are formulated with highly-effective synthetic surfactants that exhibit a high foam and primarily clean, as opposed to conditioning the hair. Therefore, many shampoos neither help detangle wet hair nor impart any residual hair conditioning benefits to dry hair, such as the manageability or styleability of hair sets. Consequently, after shampooing, the hair normally is left in a cosmetically-unsatisfactory state because an anionic surfactant-based hair shampoo composition not only removes all of the dirt and soil from the hair, but also removes essentially all of the sebum that is naturally present on the surface of the hair fibers. While conditioner compositions have been formulated to combat the challenges raised when shampooing, these conditioner compositions do not balance all of the desired characteristic with respect to combing, softness, bulk, and fly away. Therefore, the need exists for a new personal care product that can provide the desired conditioning performance to hair. WO 2009/006218 A2 discloses personal care dissolvable films comprising a water soluble film forming agent, cosmetically acceptable plasticizer, and detackifying agent, along with methods of using the same. DE 20 2016 007 571 U1 discloses a cosmetic composition in the form of a hair mousse with a conditioning effect and simultaneously good setting properties. DE 10 2010 027 487 A1 discloses hair conditioning agent with shaping properties.

### BRIEF SUMMARY

The present invention refers to a water-dissolvable film having a non-fibrous body, the non-fibrous body comprising: a quarternary salt; a polymer present in an amount ranging from 30 wt. % to 55 wt. % based on the total weight of the non-fibrous body; and a fragrance, wherein the polymer is hydroxypropyl methylcellulose; wherein the non-fibrous body is anhydrous; and wherein the non-fibrous body further comprises a starch present in an amount ranging from 5.0 wt. % to 15.0 wt. % based on the total weight of the non-fibrous body as defined in the claims.

Other embodiments of the present invention include a method for cleaning a body surface comprising applying the water-dissolvable film according to any foregoing claim to a body surface of a subject in need thereof as defined in the claims.

Generally, a method of forming a personal care product can be comprising: a) mixing together a quarternary salt, a polymer, and a fragrance to form a liquid blend; and b) processing the liquid blend into a non-fibrous body that is a water-dissolvable film.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention which is defined in the claims.

### BRIEF DESCRIPTION OF THE DRAWINGS

The present invention will become more fully understood from the detailed description and the accompanying drawings, wherein:
FIG. 1 is a perspective view of a film according to the present invention;
FIG. 2 is a cross-sectional view of the film of FIG. 1 along line X-X; and
FIG. 3 depicts a personal care product containing exemplary films of the present invention.

### DETAILED DESCRIPTION

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The description of illustrative embodiments according to principles of the present invention is intended to be read in connection with the accompanying drawings, which are to be considered part of the entire written description. In the description of embodiments of the invention disclosed herein, any reference to direction or orientation is merely intended for convenience of description and is not intended in any way to limit the scope of the present invention.

Relative terms such as "lower" , "upper," "horizontal," "vertical," "above", "below," "up," "down," "top," and "bottom" as well as derivatives thereof (e.g., "horizontally," "downwardly," "upwardly," etc.) should be construed to refer to the orientation as then described or as shown in the drawing under discussion. These relative terms are for convenience of description only and do not require that the apparatus be constructed or operated in a particular orientation unless explicitly indicated as such.

Unless otherwise specified, all percentages and amounts expressed herein and elsewhere in the specification should be understood to refer to percentages by weight. The amounts given are based on the active weight of the material. According to the present application, the term "about" means +/- 5% of the reference value. According to the present application, the term "substantially free" less than about 0.1 wt. % based on the total of the referenced value.

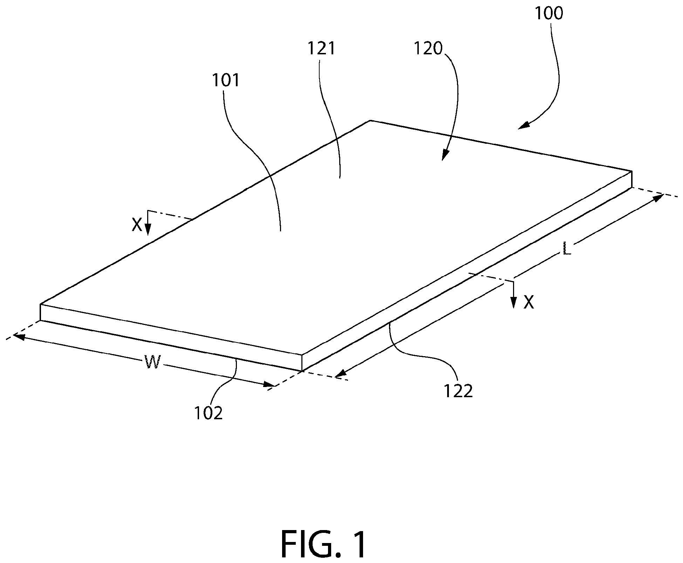

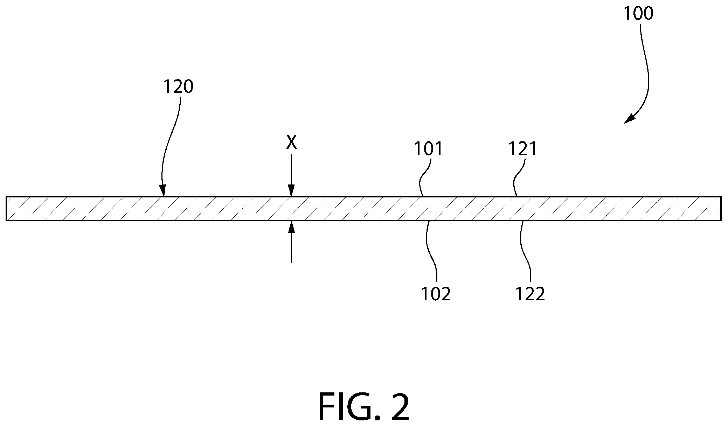

Referring now to FIGS. 1 and 2, the present invention is directed to a film 100 as defined in the claims that may have a first exposed major surface 101 opposite a second exposed major surface 102 and a side exposed surface extending there-between. The film 100 may have a film thickness t as measured between the first exposed major surface 101 and the second exposed major surface 102, the film thickness that ranges from about 1 micron to about 100 microns, including all thicknesses and sub-ranges there-between. A thickness of the film formed may vary widely. In at least one implementation, the film may have a thickness greater than or equal to about 10 µm and less than or equal to about 60 µm.

The film 100 may have a width W that ranges from about 1 micron (µm) to about 50 millimeters (mm) - including all widths and sub-ranges there-between. The film 100 may have a length L that ranges from about 5 µm to about 100 mm - including all lengths and sub-ranges there-between.

The first exposed major surface 101 may have a first surface area that ranges from about 5 µm² to about 500 cm² - including all surface areas and sub-ranges there-between. The second exposed major surface 102 may have a second surface area that ranges from about 10 µm² to about 250 cm² - including all surface areas and sub-ranges there-between. The first surface area and the second surface area may be equal.

The film 100 of the present invention may be a flexible film. The term "flexible" refers to the ability of a material or structure to be deformed under the application of stress and substantially return to its original state when the applied stress is removed. The film 100 of the present invention also forms a self-supporting structure in that the film 100 may be able to retain its structure without a separate substrate - as compared to a coating which requires a separate substrate.

The film 100 of the present invention is water-dissolvable as defined in the claims. The term "water-dissolvable" refers to the film 100 of the present invention will physically break down when exposed to liquid water such that the self-supporting structure of the film 100 ceases to exist.

The film 100 of the present invention has a body 120 that is non-fibrous as defined in the claims (also referred to as "non-fibrous body" 120). The non-fibrous body 120 may comprise a first major surface 121 opposite a second major surface 122 and a side surface extending there-between. The non-fibrous body 120 is substantially free of fiber (also referred to as fibrous material or filament). In some embodiments, the non-fibrous body 120 is entirely free of fiber (also referred to as fibrous material or filament) - i.e., 0.0 wt. % based on the total weight of the non-fibrous body 120.

The first exposed major surface 101 of the film 100 may be formed by the first major surface 121 of the non-fibrous body 120. The second exposed major surface 102 of the film 100 may be formed by the second major surface 122 of the non-fibrous body 120. The side exposed surface of the film 100 may be formed by the side surface of the non-fibrous body 120. The film 100 may be formed entirely by the non-fibrous body 120.

The non-fibrous body 120 of the present invention may be flexible. The non-fibrous body 120 also forms a self-supporting structure in that the non-fibrous body 120 may be able to retain its structure without a separate substrate. The non-fibrous body 120 may be water-dissolvable.

The non-fibrous body 120 may be formed by a composition comprising a binder and a surfactant. The body 120 may further comprise a plasticizer. The body 120 is anhydrous as defined in the claims.

The binder may be a polymeric binder. The water-dissolvable film as defined in the claims comprises a polymer present in an amount ranging from 30 wt. % to 55 wt. % based on the total weight of the non-fibrous body. The polymeric binder is present in an amount ranging from about 30 wt. % to about 55 wt. % - based on the total weight of the non-fibrous body 120 - including all percentages and sub-ranges there-between. In some embodiments, the polymeric binder may be present in an amount ranging from about 35 wt. % to about 50 wt. % - based on the total weight of the non-fibrous body 120 - including all percentages and sub-ranges there-between. In some embodiments, the polymeric binder may be present in an amount ranging from about 40 wt. % to about 45 wt. % - based on the total weight of the non-fibrous body 120 - including all percentages and sub-ranges there-between.

The polymeric binder may be a viscoelastic polymer. The polymeric binder may be a water-soluble polymer - such as hydroxyl polymer. Non-limiting examples of water-soluble hydroxyl polymers include polyols, such as polyvinyl alcohol, polyvinyl alcohol derivatives, polyvinyl alcohol copolymers, starch, starch derivatives, starch copolymers, chitosan, chitosan derivatives, chitosan copolymers, cellulose derivatives such as cellulose ether and ester derivatives, cellulose copolymers, hemicellulose, hemicellulose derivatives, hemicellulose copolymers, gums, arabinans, galactans, proteins and various other polysaccharides and mixtures thereof.

The water-dissolvable film as defined in the claims comprises a polymer which is hydroxypropylmethylcellulose.

Generally, water-soluble polymers may be or include, but are not limited to, cellulose ethers, methacrylates, polyvinylpyrollidone, or the like, and combinations thereof. For example, the water soluble polymer may include one or more cellulose ethers or cellulose polymers, selected from one or more of hydroxyalkyl cellulose polymers, such as hydroxypropyl methylcellulose (HPMC), hydroxyethylpropyl cellulose (HEPC), hydroxybutyl methyl cellulose (HBMC), hydroxypropyl cellulose, hydroxyethyl cellulose, methyl cellulose, carboxymethyl cellulose (CMC), or combinations thereof. In at least one implementation, the hydroxyalkyl cellulose polymer is a low viscosity hydroxylpropyl methyl cellulose polymer (HPMC). For example, the HPMC polymer may have a viscosity of from about 1 millipascal seconds (mPa.s) to about 100 mPa.s, as determined as a 2 wt% aqueous solution of the HPMC at 20°C using a Ubbelohde tube viscometer. In a typical implementation, the HPMC has a viscosity of about 3 mPa.s to about 20 mPa.s at 20°C. In at least one implementation, the personal care composition or the water hydratable solid thereof may include two or more hydroxyalkyl cellulose polymers having different molecular weights. In at least one implementation, the two or more hydroxyalkyl cellulose polymers may be or include, but is not limited to, METHOCEL_{TM} E5 PREMIUM LV, which is commercially available from The Dow Chemical Company of Midland, MI, Hypromellose (hydroxypropyl methylcellulose, 50 mPa.s USP Substitution Type 2910), which is commercially available from VWR International Co. of Radnor, PA, or combinations thereof. METHOCEL_{TM} E5 LV is a USP grade, low viscosity HPMC having 29.1% methoxyl groups and 9% hydroxyproxyl group substitutions. In a 2 wt% solution, the METHOCEL_{TM} has a viscosity of 5.1 mPa.s at 20°C as measured with a Ubbelohde tube viscometer.

The water-dissolvable film as defined in the claims comprises a quarternary salt. In some embodiments, the surfactant may comprise a cationic surfactant. The cationic surfactant may comprise a quarternary salt. The quarternary salt may be an esterquat. In some embodiments, the cationic surfactant may be a cationic polymer comprising a plurality of quarternary salts groups - herein referred to as a polyquat.

The esterquat may have the following formula: 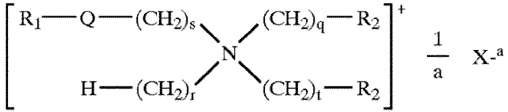 wherein Q represents a carboxyl group having the structure -OCO- or - COO-; R₁ represents an aliphatic hydrocarbon group having from 8 to 22 carbon atoms; R₂ represents -Q-R₁ or -OH; q, r, s and t, each independently represent a number of from 1 to 3; and X-a is an anion of valence a.

The cationic surfactant may be present in an amount ranging from about 0.5 wt. % to about 6.0 wt. % - based on the total weight of the non-fibrous body 120 - including all percentages and sub-ranges there-between. In some embodiments, the cationic surfactant may be present in an amount ranging from about 1.0 wt. % to about 5.0 wt. % - based on the total weight of the non-fibrous body 120 - including all percentages and sub-ranges there-between. In some embodiments, the cationic surfactant may be present in an amount ranging from about 2.0 wt. % to about 4.0 wt. % - based on the total weight of the non-fibrous body 120 - including all percentages and sub-ranges there-between.

According to the present invention, it has been discovered that by using a cationic surfactant - specifically, an esterquat - the film 100 may impart superior condition properties when added to a personal care product, such as a hair conditioning composition. Specifically, the film 100 may impart satisfactory-to-superior combing (both dry and wet), hair-softness, hair-bulk, and fly away.

The surfactant may further comprise a non-ionic surfactant. The surfactant may further comprise a non-ionic surfactant such that the non-fibrous body 120 comprises both a cationic surfactant and a non-ionic surfactant. The non-ionic surfactant may be a polysorbate compound having the formula: 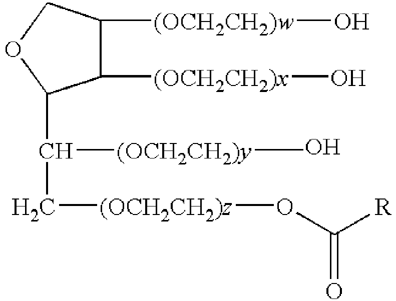 wherein w, x, and y is respectively an integer of 0-20, preferably 1-20, and z is an integer of 1-20, and wherein w + x + y + z = 4-30 - including all integers and sub-ranges there-between. In some embodiments, w + x + y + z = 4-25 - including all integers and sub-ranges there-between. In some embodiments, w + x + y + z = 10-25 - including all integers and sub-ranges there-between. The group R may be linear or branched, and saturated or unsaturated alkyl having 11-23 carbon atoms, preferably from 12 to 18 carbon atoms. In some embodiments, the polysorbate compound may be polysorbate 80.

The cationic surfactant and the non-ionic surfactant may be present in a weight ratio ranging from about 1.5:1 to about 2.5:1 - including all ratios and sub-ranges there-between. In some embodiments, the cationic surfactant and the non-ionic surfactant may be present in a weight ratio ranging of about 2:1.

The non-fibrous body 120 may further comprise a plasticizer. The plasticizer may be selected from one or more of diols, polyols, copolyols, polycarboxylic acids, polyesters and dimethicone copolyols. Non-limiting examples of diol / polyols include glycerin, diglycerin, propylene glycol, ethylene glycol, butylene glycol, pentylene glycol, cyclohexane dimethanol, hexanediol, 2,2,4-trimethylpentane-1,3-diol, polyethylene glycol (200-600), pentaerythritol, sugar alcohols such as sorbitol, mannitol, lactitol and other mono- and polyhydric low molecular weight alcohols (e.g., C2-C8 alcohols); mono di- and oligo-saccharides such as fructose, glucose, sucrose, maltose, lactose, high fructose corn syrup solids, and dextrins, and ascorbic acid.

In some embodiments, the plasticizer includes glycerin and/or propylene glycol and/or glycerol derivatives such as propoxylated glycerol. In another example, the plasticizer is selected from the group consisting of glycerin, ethylene glycol, polyethylene glycol, propylene glycol, glycidol, urea, sorbitol, xylitol, maltitol, sugars, ethylene bisformamide, amino acids, and mixtures thereof.

The plasticizer may be present in an amount ranging from about 1.0 wt. % to about 8.0 wt. % - based on the total weight of the non-fibrous body 120 - including all percentages and sub-ranges there-between. In some embodiments, the plasticizer may be present in an amount ranging from about 2.0 wt. % to about 7.0 wt. % - based on the total weight of the non-fibrous body 120 - including all percentages and sub-ranges there-between. In some embodiments, the plasticizer may be present in an amount ranging from about 3.0 wt. % to about 6.0 wt. % - based on the total weight of the non-fibrous body 120 - including all percentages and sub-ranges there-between.

The non-fibrous body 120 of the present invention may further comprise one or more colorants. The colorants may be a pigment, a dye, or mixtures thereof. For example, metal oxide pigments, those which are surface treated titanium dioxide, optionally, zirconium oxide or cerium oxide, zinc oxide, iron oxide (black, yellow or red), chromium oxide, manganese violet, ultramarine blue, chromium hydrate and ferric blue, carbon black, barium, strontium, calcium or aluminum pigment (e.g. D & C or FD & C), cochineal carmine, oxy titanium or bismuth mica coated with chloride, (those with iron oxide) of titanium mica, titanium mica (particularly those involving ferric blue or chromium oxide), (those with organic pigments) titanium mica, bismuth oxy a pearl pigment those that chloride to the group, Goniokuroma Click (goniochromatic) pigments. Other examples include pigments with a multilayer interference structure, reflective pigments, such as silver coated glass materials, nickel / chromium / glass material coated with molybdenum alloy, a glass substrate coated with brown iron oxide particles having a particle comprising a stack of at least two polymer layers, for example (from 3M) MIRROR GLITTER, and the like.

Examples of the dye include water-soluble dyes such as second copper sulfate, ferrous sulfate, water-soluble sulfopolyesters, natural dyes, for example carotene and beet juice roots, methylene blue, caramel, tartrazine disodium salt and fustin (fuschin) sodium salt, and mixtures thereof. Lipophilic dyes may also optionally be used. Additional non-limiting examples of dyes include food dyes suitable for food, drug and cosmetic applications, and mixtures thereof.

The colorants may be present in an amount ranging from about 0.1 wt. % to about 4.0 wt. % - based on the total weight of the non-fibrous body 120 - including all percentages and sub-ranges there-between. In some embodiments, the colorants may be present in an amount ranging from about 0.5 wt. % to about 3.0 wt. % - based on the total weight of the non-fibrous body 120 - including all percentages and sub-ranges there-between. In some embodiments, the colorants may be present in an amount ranging from about 1.0 wt. % to about 2.0 wt. % - based on the total weight of the non-fibrous body 120 - including all percentages and sub-ranges there-between.

The non-fibrous body further comprises a starch present in an amount ranging from 5.0 wt. % to 15.0 wt. % based on the total weight of the non-fibrous body. The starch may be a natural starch.

Naturally occurring starch is generally a mixture of linear amylose and branched amylopectin polymer of D-glucose units. The amylose is a substantially linear polymer of D-glucose units joined by (1,4)-α-D links. The amylopectin is a highly branched polymer of D-glucose units joined by (1,4)-α-D links and (1,6)-α-D links at the branch points. Naturally occurring starch typically contains relatively high levels of amylopectin, for example, corn starch (64-80% amylopectin), waxy maize (93-100% amylopectin), rice (83-84% amylopectin), potato (about 78% amylopectin), and wheat (73-83% amylopectin). Though all starches are potentially useful herein, the present invention is most commonly practiced with high amylopectin natural starches derived from agricultural sources, which offer the advantages of being abundant in supply, easily replenishable and inexpensive.

As used herein, "starch" includes any naturally occurring unmodified starches, modified starches, synthetic starches and mixtures thereof, as well as mixtures of the amylose or amylopectin fractions; the starch may be modified by physical, chemical, or biological processes, or combinations thereof. The choice of unmodified or modified starch for the present invention may depend on the end product desired. In one embodiment of the present invention, the starch or starch mixture useful in the present invention has an amylopectin content from about 20% to about 100%, more typically from about 40% to about 90%, even more typically from about 60% to about 85% by weight of the starch or mixtures thereof.

Suitable naturally occurring starches can include, but are not limited to, corn starch, potato starch, sweet potato starch, wheat starch, sago palm starch, tapioca starch, rice starch, soybean starch, arrow root starch, amioca starch, bracken starch, lotus starch, waxy maize starch, and high amylose corn starch. In a preferred embodiment, the starch is corn starch.

The starch is present in an amount ranging from about 5.0 wt. % to about 15.0 wt. % - based on the total weight of the non-fibrous body 120 - including all percentages and sub-ranges there-between. In some embodiments, the starch may be present in an amount ranging from about 7.0 wt. % to about 13.0 wt. % - based on the total weight of the non-fibrous body 120 - including all percentages and sub-ranges there-between. In some embodiments, the starch may be present in an amount ranging from about 9.0 wt. % to about 11.0 wt. % - based on the total weight of the non-fibrous body 120 - including all percentages and sub-ranges there-between.

The non-fibrous body 120 may further comprise one or more fragrances or perfume. The fragrance and/or perfume may be present in an amount ranging from about 25.0 wt. % to about 50.0 wt. % - based on the total weight of the non-fibrous body 120 - including all percentages and sub-ranges there-between. In some embodiments, the fragrance and/or perfume may be present in an amount ranging from about 30.0 wt. % to about 45.0 wt. % - based on the total weight of the non-fibrous body 120 - including all percentages and sub-ranges there-between. In some embodiments, the fragrance and/or perfume may be present in an amount ranging from about 35.0 wt. % to about 40.0 wt. % - based on the total weight of the non-fibrous body 120 - including all percentages and sub-ranges there-between.

Non-limiting examples of fragrances and perfumes include odor compounds selected from: 7-acetyl-1,2,3,4,5,6,7,8-octahydro-1,1,6,7-tetramethylnaphthalene, α-ionone, β-ionone, γ-ionone α-isomethylionone, methylcedrylone, methyl dihydrojasmonate, methyl 1,6,10-trimethyl-2,5,9-cyclododecatrien-1-yl ketone, 7-acetyl-1,1,3,4,4,6-hexamethyltetralin, 4-acetyl-6-tert-butyl-1,1-dimethylindane, hydroxyphenylbutanone, benzophenone, methyl β-naphthyl ketone, 6-acetyl-1,1,2,3,3,5-hexamethylindane, 5-acetyl-3-isopropyl-1,1,2,6-tetramethylindane, 1-dodecanal, 4-(4-hydroxy-4-methylpentyl)-3-cyclohexene-1-carboxaldehyde, 7-hydroxy-3,7-dimethyloctanal, 10-undecen-1-al, isohexenylcyclohexylcarboxaldehyde, formyltricyclodecane, condensation products of hydroxycitronellal and methyl anthranilate, condensation products of hydroxycitronellal and indole, condensation products of phenylacetaldehyde and indole, 2-methyl-3-(para-tert-butylphenyl)propionaldehyde, ethylvanillin, heliotropin, hexylcinnamaldehyde, amylcinnamaldehyde, 2-methyl-2-(isopropylphenyl)propionaldehyde, coumarin, γ-decalactone, cyclopentadecanolide, 16-hydroxy-9-hexadecenoic acid lactone, 1,3,4,6,7,8-hexahydro-4,6,6,7,8,8-hexamethylcyclopenta-γ-2-benzopyran, β-naphthol methyl ether, ambroxane, dodecahydro-3*a*,6,6,9*a*-tetramethylnaphtho[2,1*b*]furan, cedrol, 5-(2,2,3-trimethylcyclopent-3-enyl)-3-methylpentan-2-ol, 2-ethyl-4-(2,2,3-trimethyl-3-cyclopenten-1-yl)-2-buten-1-ol, caryophyllene alcohol, tricyclodecenyl propionate, tricyclodecenyl acetate, benzyl salicylate, cedryl acetate, and tert-butylcyclohexyl acetate.

Other fragrances may include odor compounds selected from essential oils, resinoids and resins from a large number of sources, such as, for example, Peru balsam, olibanum resinoid, styrax, labdanum resin, nutmeg, cassia oil, benzoin resin, coriander, and lavandin. Further suitable fragrances include odor compounds selected from phenylethyl alcohol, terpineol, linalool, linalyl acetate, geraniol, nerol, 2-(1,1-dimethylethyl)cyclo-hexanol acetate, benzyl acetate, and eugenol.

The fragrances or perfumes can be used as single substances or in a mixture with one another.

In some embodiments, the fragrance may be provided as a microcapsule formulation, whereby the odor-compound is surrounded by a release controlling membrane, which may serve as a protective shell. The material inside the microcapsule is referred to as the core, internal phase, or fill, whereas the wall is sometimes called a shell, coating, or membrane.

The possible shell materials vary widely in their stability toward water. Among the most stable are polyoxymethyleneurea (PMU)-based materials, which may hold certain PRMs for even long periods of time in aqueous solution (or product). Such systems include but are not limited to urea-formaldehyde and/or melamine-formaldehyde.

Stable shell materials include polyacrylate-based materials obtained as reaction product of an oil soluble or dispersible amine with a multifunctional acrylate or methacrylate monomer or oligomer, an oil soluble acid and an initiator, in presence of an anionic emulsifier comprising a water soluble or water dispersible acrylic acid alkyl acid copolymer, an alkali or alkali salt. Gelatin-based microcapsules may be prepared so that they dissolve quickly or slowly in water, depending for example on the degree of cross-linking.

Many other capsule wall materials are available and vary in the degree of perfume diffusion stability observed. Without wishing to be bound by theory, the rate of release of perfume from a capsule, for example, once deposited on a surface is typically in reverse order of in-product perfume diffusion stability. As such, urea-formaldehyde and melamine-formaldehyde microcapsules for example, typically require a release mechanism other than, or in addition to, diffusion for release, such as mechanical force (e.g., friction, pressure, shear stress) that serves to break the capsule and increase the rate of perfume (fragrance) release.

The odor compound may be included as part of a microencapsulated fragrance or perfume or a non-encapsulated fragrance or perfume.

The film 100 of the present invention may be formed by mixing together the binder (e.g., polymeric binder), the surfactant, and the fragrance to form a blend. In some embodiments, the film 100 of the present invention may be formed by mixing together the binder (.e.g, polymeric binder), the surfactant, the plasticizer, the starch, and the fragrance to form a blend. The blend may be a liquid. The blend may then be processing into a sheet, which is then cut into multiples of the non-fibrous body 120, whereby each non-fibrous body 120 forms the film 100.

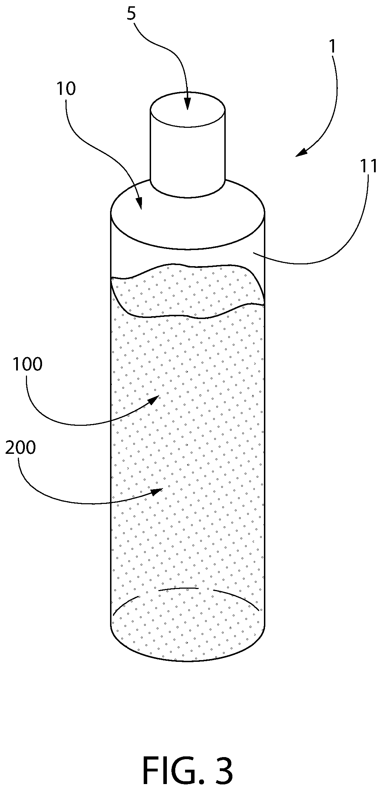

Referring now to FIG. 3, the present invention further includes a personal care composition 200 that comprises the film 100 of the present invention. The present invention further comprises a personal care product 1 comprising the personal care composition of the present invention.

The personal care composition 200 may be a hair-conditioning composition, a hair shampoo composition, and the like. The personal care composition 200 of the present invention may further comprise an additional components suitable for use with a hair-conditioning and/or hair-shampoo composition.

The personal care product 1 of the present invention may include a container 10 comprising a reservoir 11 where the personal care composition 200 may be located. The container 10 may further comprise an opening through which the personal care composition 200 may be dispensed out of from the reservoir 11.

The personal care product 1 may further comprise a cap 5 which may seal the opening of the container to seal the reservoir 11, thereby preventing any personal care product 200 from inadvertently escaping the reservoir 11.

While the invention has been described with respect to specific examples including presently preferred modes of carrying out the invention, those skilled in the art will appreciate that there are numerous variations and permutations of the above described systems and techniques. It is to be understood that other embodiments may be utilized and structural and functional modifications may be made without departing from the scope of the present invention. Thus, the scope of the invention is defined in the claims.

### EXAMPLES

An experiment was performed to test the conditioning performance of the film of the present invention. Two film formulations were prepared - as set forth below in Table 1.

**Table 1**

| | Comparative Example 1 | Example 1 |
|---|---|---|
| Hydroxypropyl Methylcellulose | 44.0 | 42.6 |
| Melamine-Formaldehyde Encapsulated Fragrance | 37.3 | 36.1 |
| Esterquat | - | 3.3 |
| Propylene Glycol | 5.1 | 4.9 |
| Titanium Dioxide | 1.7 | 1.6 |
| Polysorbate 80 | 1.7 | 1.6 |
| Corn Starch | 10.2 | 9.9 |
| | | |
| Total | 100.0 | 100.0 |

Each example was then tested for wet combing, dry combing, and softness. Two (2) sets of five (5) hair tresses each are prepared and cleaned with 20% solution of sodium laureth sulfate. Each set of hair tresses is washed with 1 mL of a commercially available shampoo, and 0.1 g of either an exemplary composition of the present invention or a comparative composition, per hair tress. After each treatment, trained in-house expert panelists, blindly evaluate the tresses. Tresses are randomly assigned to avoid bias. Panelist evaluate both wet and dry stages. Wet stage evaluation is more relevant for describing hair conditioning benefit. This relates to ease of combing. The best performer are assigned a score of two points and the low performer assigned a score of one point.

Hair volume and alignment evaluations are carried out with Rumba equipment from Bossa Nova-Tech. Two (2) sets of six (6) hair tresses are prepared and cleaned with twenty percent (20%) solution of sodium laureth sulfate. Each set is then treated with 0.5 mL of a commercially available shampoo and 0.1 g of either an exemplary composition of the present invention or a comparative composition, per hair tress. The Rumba equipment takes pictures of a hair tress suspended using a clamp while illuminating the sample with polarized light in multiple orientations. The pictures are processed with Rumba software to determine hair orientation using a color index. The comparison of colored pixels on each image with the non-reflective background surface is used to measure the volume of the hair tress. The software is also used to measure hair volume and alignment. The results of these experiments are described below in Table 2.

**Table 2**

| | Comparative Example 1 | Example 1 |
|---|---|---|
| Wet Combing | 1.200 | 1.800 |
| Dry Combing | 1.500 | 1.500 |
| Softness | 1.400 | 1.600 |
| Volume Bulk | 154417 | 174016 |
| Alignment Coefficient | 28.22 | 35.56 |

As demonstrated by the data described in Table 2 (above), exemplary films of the present invention provide an unexpected improvement in wet combing, as well as enhancement of softness, volume, and hair alignment without any detrimental impact on dry combing performance.

## Claims

1. A water-dissolvable film having a non-fibrous body, the non-fibrous body comprising:
a quarternary salt;
a polymer present in an amount ranging from 30 wt. % to 55 wt. % based on the total weight of the non-fibrous body; and
a fragrance;
wherein the polymer is hydroxypropyl methylcellulose;
wherein the non-fibrous body is anhydrous; and
wherein the non-fibrous body further comprises a starch present in an amount ranging from 5.0 wt. % to 15.0 wt. % based on the total weight of the non-fibrous body.

2. The water-dissolvable film according to any claim 1, wherein the quarternary salt comprises an esterquat.

3. The water-dissolvable film according to any foregoing claim, wherein the quarternary salt comprises a polyquaternium salt.

4. The water-dissolvable film according to any foregoing claim, wherein the quarternary salt is present in an amount ranging from 0.5 wt. % to 6.0 wt. % based on the total weight of the non-fibrous body.

5. The water-dissolvable film according to any foregoing claim, further comprises a plasticizer selected from a diol, a polyol, and combinations thereof; optionally
wherein the plasticizer comprises a diol; and optionally
wherein the diol is selected from the group consisting of propylene glycol, ethylene glycol, butane-1,4 diol, isobutene-1,3 diol; and a combination of two or more thereof; and/or
wherein the plasticizer is present in an amount ranging from 1.0 wt. % to 8.0 wt. % based on the total weight of the non-fibrous body.

6. The water-dissolvable film according to any foregoing claim, wherein the non-fibrous body further comprises a non-ionic surfactant; optionally
wherein the non-ionic surfactant is selected from the group consisting of polysorbate 80, polysorbate 20 polysorbate 60; and a combination of two or more thereof; and optionally
wherein the non-ionic surfactant is present in an amount ranging from 1.0 wt. % to 3.0 wt. % based on the total weight of the non-fibrous body.

7. The water-dissolvable film according to any foregoing claim, wherein the non-fibrous body further comprises a colorant; optionally
wherein the colorant is selected from: titanium dioxide; zinc oxide; a pigment; a dye; and a combination of two or more thereof; and optionally
wherein the colorant is present in an amount ranging from 0.1 wt. % to 3.0 wt. % based on the total weight of the non-fibrous body.

8. The water-dissolvable film according to any foregoing claim, wherein the fragrance is selected from a melamine-formaldehyde encapsulated fragrance; a urea-formaldehyde encapsulated fragrance; and a combination thereof.

9. The water-dissolvable film according to any foregoing claim, wherein the fragrance is present in an amount ranging from 30.0 wt. % to 50.0 wt. % based on the total weight of the non-fibrous body.

10. The water-dissolvable film according to any foregoing claim,
wherein the starch is selected from: corn starch; tapioca starch; potato starch; wheat starch; rice starch; a starch derivative; and a combination of two or more.

11. A method for cleaning a body surface comprising applying the water-dissolvable film according to any foregoing claim to a body surface of a subject in need thereof.

12. The method according to claim 11, wherein the body surface comprises a keratinous substance; optionally
wherein the keratinous substance is selected from hair; skin; and nails.

## Patentansprüche

1. Wasserlöslicher Film, aufweisend einen nicht-faserigen Körper, wobei der nicht-faserige Körper aufweist:
ein quaternäres Salz;
ein Polymer, das in einer Menge von 30 Gew.-% bis 55 Gew.-%, bezogen auf das Gesamtgewicht des nicht-faserigen Körpers, vorliegt; und
einen Duftstoff;
wobei das Polymer Hydroxypropylmethylcellulose ist;
wobei der nicht-faserige Körper wasserfrei ist; und
wobei der nicht-faserige Körper ferner eine Stärke aufweist, die in einer Menge von 5,0 Gew.-% bis 15,0 Gew.-%, bezogen auf das Gesamtgewicht des nicht-faserigen Körpers, vorliegt.

2. Wasserlöslicher Film nach Anspruch 1, wobei das quaternäre Salz ein Esterquat aufweist.

3. Wasserlöslicher Film nach einem der vorhergehenden Ansprüche, wobei das quaternäre Salz ein Polyquaternium-Salz aufweist.

4. Wasserlöslicher Film nach einem der vorhergehenden Ansprüche, wobei das quaternäre Salz in einer Menge von 0,5 Gew.-% bis 6,0 Gew.-%, bezogen auf das Gesamtgewicht des nicht-faserigen Körpers, vorliegt.

5. Wasserlöslicher Film nach einem der vorhergehenden Ansprüche, wobei der nicht-faserige Körper ferner einen Weichmacher aufweist, der aus einem Diol, einem Polyol und Kombinationen davon ausgewählt ist; optional
wobei der Weichmacher ein Diol aufweist; und optional
wobei das Diol aus der Gruppe ausgewählt ist, die aus Propylenglykol, Ethylenglykol, Butan-1,4-diol, Isobuten-1,3-diol und einer Kombination von zwei oder mehr davon besteht; und/oder
wobei der Weichmacher in einer Menge von 1,0 Gew.-% bis 8,0 Gew.-%, bezogen auf das Gesamtgewicht des nicht-faserigen Körpers, vorliegt.

6. Wasserlöslicher Film nach einem der vorhergehenden Ansprüche, wobei der nicht-faserige Körper ferner ein nichtionisches Tensid aufweist; optional
wobei das nichtionische Tensid aus der Gruppe ausgewählt ist, die aus Polysorbat 80, Polysorbat 20, Polysorbat 60 und einer Kombination von zwei oder mehr davon besteht; und optional
wobei das nichtionische Tensid in einer Menge von 1,0 Gew.-% bis 3,0 Gew.-%, bezogen auf das Gesamtgewicht des nicht-faserigen Körpers, vorliegt.

7. Wasserlöslicher Film nach einem der vorhergehenden Ansprüche, wobei der nicht-faserige Körper ferner einen Farbstoff aufweist; optional
wobei der Farbstoff ausgewählt ist aus: Titandioxid; Zinkoxid; einem Pigment; einem Farbstoff; und einer Kombination von zwei oder mehr davon; und optional
wobei der Farbstoff in einer Menge von 0,1 Gew.-% bis 3,0 Gew.-%, bezogen auf das Gesamtgewicht des nicht-faserigen Körpers, vorliegt.

8. Wasserlöslicher Film nach einem der vorhergehenden Ansprüche, wobei der Duftstoff aus einem Melamin-Formaldehyd-verkapselten Duftstoff, einem Harnstoff-Formaldehyd-verkapselten Duftstoff und einer Kombination davon ausgewählt ist.

9. Wasserlöslicher Film nach einem der vorhergehenden Ansprüche, wobei der Duftstoff in einer Menge von 30,0 Gew.-% bis 50,0 Gew.-%, bezogen auf das Gesamtgewicht des nicht-faserigen Körpers, vorliegt.

10. Wasserlöslicher Film nach einem der vorhergehenden Ansprüche,
wobei die Stärke ausgewählt ist aus: Maisstärke; Tapiokastärke; Kartoffelstärke; Weizenstärke; Reisstärke; einem Stärkederivat; und einer Kombination von zwei oder mehr davon.

11. Verfahren zum Reinigen einer Körperoberfläche, aufweisend das Aufbringen des wasserlöslichen Films nach einem der vorhergehenden Ansprüche auf eine Körperoberfläche eines Subjekts, das dessen bedarf.

12. Verfahren nach Anspruch 11, wobei die Körperoberfläche eine keratinische Substanz aufweist; optional
wobei die keratinische Substanz aus Haaren, Haut und Nägeln ausgewählt ist.

## Revendications

1. Film soluble dans l'eau comportant un corps non fibreux, le corps non fibreux comprenant :
un sel quaternaire ;
un polymère présent en une quantité allant de 30 % en poids à 55 % en poids par rapport au poids total du corps non fibreux ; et
un parfum ;
dans lequel le polymère est l'hydroxypropylméthylcellulose ;
dans lequel le corps non fibreux est anhydre ; et
dans lequel le corps non fibreux comprend en outre un amidon présent en une quantité allant de 5,0 % en poids à 15,0 % en poids par rapport au poids total du corps non fibreux.

2. Film soluble dans l'eau selon l'une quelconque de la revendication 1, dans lequel le sel quaternaire comprend un esterquat.

3. Film soluble dans l'eau selon l'une quelconque des revendications précédentes, dans lequel le sel quaternaire comprend un sel de polyquaternium.

4. Film soluble dans l'eau selon l'une quelconque des revendications précédentes, dans lequel le sel quaternaire est présent en une quantité allant de 0,5 % en poids à 6,0 % en poids par rapport au poids total du corps non fibreux.

5. Film soluble dans l'eau selon l'une quelconque des revendications précédentes, comprenant en outre un plastifiant choisi parmi un diol, un polyol et des combinaisons de ceux-ci ; éventuellement
dans lequel le plastifiant comprend un diol ; et éventuellement
dans lequel le diol est choisi dans le groupe constitué par le propylène glycol, l'éthylène glycol, le butane-1,4 diol, l'isobutène-1,3 diol ; et une combinaison de deux ou plus de ceux-ci ; et/ou
dans lequel le plastifiant est présent en une quantité allant de 1,0 % en poids à 8,0 % en poids par rapport au poids total du corps non fibreux.

6. Film soluble dans l'eau selon l'une quelconque des revendications précédentes, dans lequel le corps non fibreux comprend en outre un tensioactif non ionique ; éventuellement
dans lequel le tensioactif non ionique est choisi dans le groupe constitué par le polysorbate 80, le polysorbate 20, le polysorbate 60 ; et une combinaison de deux ou plus de ceux-ci ; et éventuellement
dans lequel le tensioactif non ionique est présent en une quantité allant de 1,0 % en poids à 3,0 % en poids par rapport au poids total du corps non fibreux.

7. Film soluble dans l'eau selon l'une quelconque des revendications précédentes, dans lequel le corps non fibreux comprend en outre un colorant ; éventuellement
dans lequel le colorant est choisi parmi : le dioxyde de titane ; l'oxyde de zinc ; un pigment ; un colorant ; et une combinaison de deux ou plus de ceux-ci ; et éventuellement
dans lequel le colorant est présent en une quantité allant de 0,1 % en poids à 3,0 % en poids par rapport au poids total du corps non fibreux.

8. Film soluble dans l'eau selon l'une quelconque des revendications précédentes, dans lequel le parfum est choisi parmi un parfum encapsulé mélamine-formaldéhyde ; un parfum encapsulé urée-formaldéhyde ; et une combinaison de ceux-ci.

9. Film soluble dans l'eau selon l'une quelconque des revendications précédentes, dans lequel le parfum est présent en une quantité allant de 30,0 % en poids à 50,0 % en poids par rapport au poids total du corps non fibreux.

10. Film soluble dans l'eau selon l'une quelconque des revendications précédentes,
dans lequel l'amidon est choisi parmi : l'amidon de maïs ; l'amidon de manioc ; l'amidon de pomme de terre ; l'amidon de blé ; l'amidon de riz ; un dérivé d'amidon ; et une combinaison de deux ou plus.

11. Procédé de nettoyage d'une surface corporelle comprenant l'application du film soluble dans l'eau selon l'une quelconque des revendications précédentes sur une surface corporelle d'un sujet en ayant besoin.

12. Procédé selon la revendication 11, dans lequel la surface corporelle comprend une substance kératinique ; éventuellement
dans lequel la substance kératinique est choisie parmi les cheveux ; la peau ; et les ongles.
